# FASCICULE DE BREVET EUROPEEN

(11) **EP 0 890 371 B1**
(45) Date de publication et mention de la délivrance du brevet: **14.01.2004**
(21) Numéro de dépôt: 98401661.8
(22) Date de dépôt: 02.07.1998
(51) Int. Cl.: A61N 1/375

(54) **Systéme d'immobilisation mécanique d'un connecteur de sonde dans un connecteur de générateur de dispositif médical implantable actif, notamment pour stimulateur cardiaque, défibrillateur et/ou cardioverteur**
Mechanisches Verriegelungssystem für die Anschlussklemme einer aktiven implantierbaren medizinischen Einrichtung, insbesondere für einen Herzstimulator, Defibrillator und/oder Kardiovertierer
Mechanical locking system for a pacing lead connector of an active implantable medical device, especially for a cardiac stimulator, defibrillator and/or cardioverter

(30) Priorité: 03.07.1997 FR 9708429
(43) Date de publication de la demande: 13.01.1999
(73) Titulaire: ELA MEDICAL (Société anonyme), 92541 Montrouge (FR)
(72) Inventeur: Correas, Philippe, 95230 Soissy Sous Montmorency (FR)
(74) Mandataire: Dupuis-Latour, Dominique

(56) Documents cités:
- WO-A-91/04069
- DE-A- 3 712 633
- US-A- 4 712 557

## Description

La présente invention concerne les dispositifs médicaux implantables actifs. Elle sera principalement décrite dans le cas d'un stimulateur cardiaque, mais il ne s'agit là que d'un exemple de mise en oeuvre de l'invention, qui est applicable de façon beaucoup plus générale à une très grande variété de "dispositifs médicaux implantables actifs" tels que définis par la Directive 90/385/CE du 20 juin 1990 du Conseil des communautés européennes, définition qui inclut, outre les stimulateurs cardiaques, les défibrillateurs et/ou cardioverteurs, les appareils neurologiques, les pompes de diffusion de substances médicales, les implants cochléaires, les capteurs biologiques implantés, etc.

Ces dispositifs comportent un boîtier généralement désigné "générateur" ou "générateur d'impulsions" raccordé électriquement et mécaniquement à une sonde, ce raccordement étant réalisé par le chirurgien au moment de l'implantation.

On pourra à cet effet se reporter à la norme française et européenne NF EN 50077 *"Connecteur à bas profil pour stimulateurs cardiaques implantables",* qui définit un système de connexion normalisé dit "IS-1" permettant de garantir l'interchangeabilité des sondes et des générateurs d'impulsions produits par différents fabricants (l'invention n'est toutefois pas limitée au cas particulier des systèmes de connexion selon cette norme, ni même aux systèmes de connexion pour stimulateurs cardiaques).

Jusqu'à présent, le raccordement entre le connecteur de la sonde et le connecteur du générateur était réalisé par une ou plusieurs vis, serrées par le chirurgien au moyen d'un outil *ad hoc* (tournevis muni éventuellement d'un limiteur de couple) au moment de l'implantation.

Ce raccordement par vis présente cependant plusieurs inconvénients. En premier lieu, outre un outil spécifique pour sa mise en oeuvre, cette technique impose la présence de bouchons de passage étanche de cet outil (pour éviter que, après implantation, les bornes de connexion ne viennent en contact avec des fluides organiques). Cette exigence d'étanchéité au niveau des fentes de passage de l'outil implique un surcoût et une augmentation de volume du générateur au niveau du connecteur.

Par ailleurs, ce système de raccordement n'est pas à l'abri d'un oubli de serrage de vis de la part du chirurgien, d'un serrage insuffisant, ou d'un serrage trop violent endommageant le dispositif.

Le document US-A-4 712 577 divulgue un système de connexion sans vis de serrage pour un dispositif médical implantable actif.

L'un des buts de l'invention est de s'affranchir de ces inconvénients, en proposant un autre système de connexion permettant d'assurer le raccordement électrique et mécanique de la sonde au générateur supprimant tout recours à une vis de serrage.

Comme on le verra, le mécanisme de l'invention permet néanmoins d'assurer une excellente immobilisation mécanique de la sonde sur le générateur, en évitant donc tout risque de déconnexion inopportune.

En outre, l'invention reste parfaitement compatible avec, notamment, le standard mécanique dimensionnel IS-1, ce qui lui permet d'accepter sans modification toutes les sondes normalisées actuelles ou à venir.

À cet effet, le dispositif médical implantable actif de l'invention, qui comprend un connecteur de générateur comportant un logement femelle axial recevant un connecteur de sonde, au moins une borne de liaison et des moyens d'immobilisation mécanique du connecteur de sonde dans le logement, la borne de liaison comportant des moyens pour exercer une pression d'appui radial d'un élément conducteur de la borne contre un élément conducteur du connecteur de sonde, et comprennent des moyens d'immobilisation distincts des bornes de liaison et, au débouché du logement, une cale de blocage présentant en section longitudinale un profil cunéiforme et propre à être insérée à force entre une surface extérieure de la sonde et une surface intérieure du logement.

Selon diverses caractéristiques subsidiaires avantageuses:
- la surface intérieure du logement comporte un évidement conique dont la surface forme une rampe homologue du profil cunéiforme de la cale de blocage ;
- la cale de blocage comporte une surface cylindrique propre à venir en contact avec la surface extérieure de la sonde et une surface conique propre à venir en contact avec la surface intérieure du logement ;
- dans un plan radial, la cale s'étend circonférentiellement sur un secteur angulaire d'ouverture comprise entre 90 et 120°;
- il est prévu des moyens d'encliquetage en position opérante de la cale dans le logement, notamment des moyens comprenant une gorge formée dans la cale coopérant avec un relief homologue formé dans le logement, ou l'inverse, gorge et relief s'étendant sur une fraction de circonférence dans un plan radial ;
- il est prévu des moyens de retenue de la cale de blocage dans le logement en l'absence de sonde, notamment des moyens comportant un ergot de butée en position proximale, coopérant avec un relief du logement en position distale ;
- les moyens de la borne de liaison pour exercer une pression d'appui radial comportent des moyens de sollicitation élastique de l'élément conducteur de la borne contre l'élément conducteur du connecteur ;
- la sonde comporte un épaulement pour solliciter axialement la cale de blocage dans le sens de l'enfoncement lors de l'insertion de la sonde dans le logement.

D'autres caractéristiques de l'invention apparaîtront à la lecture de la description détaillée ci-dessous d'un exemple de réalisation.

La figure 1 est une vue longitudinale, en coupe selon I-I de la figure 2, d'un système de connexion selon l'invention, avec un connecteur de sonde inséré dans un connecteur de générateur.

La figure 2 est une vue arrière, selon II-II de la figure 1, du système de connexion de la figure 1.

La figure 3 est une vue agrandie du détail repéré III sur la figure 1, montrant l'une des bornes de liaison.

La figure 4 est une coupe selon IV-IV de la figure 3 de ce même détail.

La figure 5 est une vue agrandie du détail repéré V sur la figure 1, montrant la forme du logement du connecteur du générateur à cet endroit.

La figure 6 est une vue perspective agrandie de la cale de blocage de la sonde dans le logement du générateur.

Sur les figures, la référence 10 désigne de façon générale la partie supérieure du générateur d'impulsions d'un stimulateur cardiaque, dans cet exemple un générateur prévu pour recevoir une sonde bipolaire, avec deux bornes de liaison 12, 14 noyées dans un corps isolant, par exemple en résine époxy. Ces bornes de liaison 12, 14 comportent des éléments conducteurs 16, 18 venant en appui contre des surfaces conductrices respectives coaxiales 20, 22, décalées axialement, de manière en elle-même classique dans le cas de sondes bipolaires, notamment de sondes conformes au standard mécanique dimensionnel IS-1 mentionné au début de la présente description.

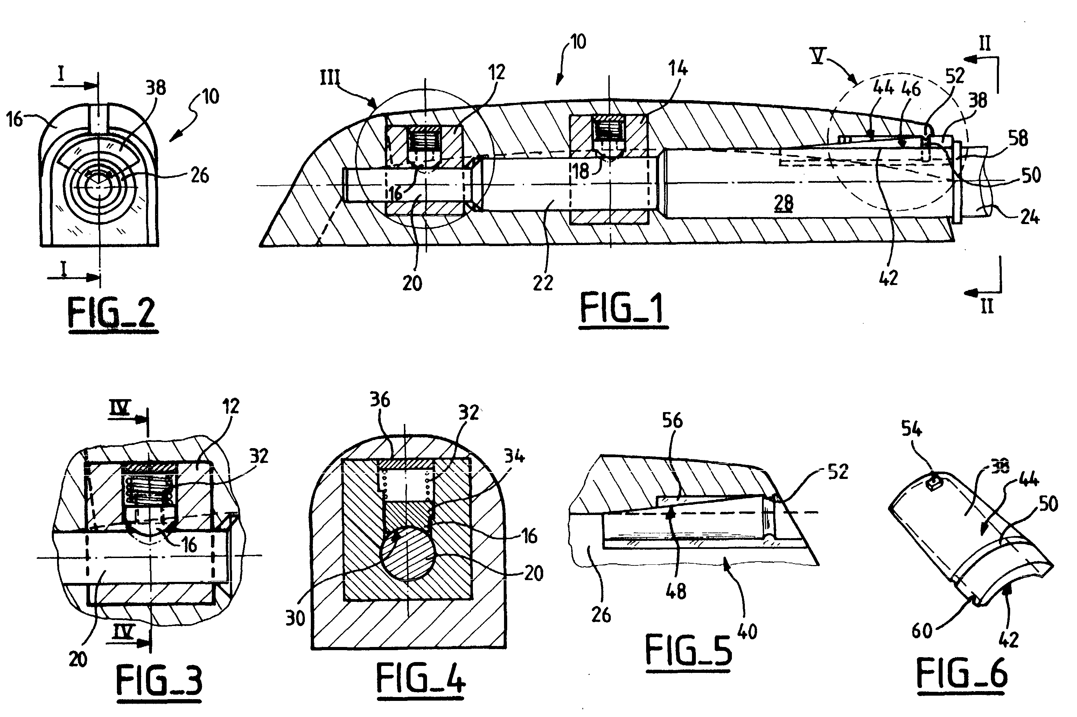

Mis à part les régions des surfaces 20, 22 destinées à établir la connexion électrique, la sonde 24 se présente extérieurement sous forme d'un corps cylindrique 28 en un matériau légèrement déformable (habituellement un polymère de silicone), qui vient se loger dans un logement homologue 26 (visible notamment figures 2 et 5, qui sont des vues sans la sonde). Ce logement 26 peut être notamment conforme aux prescriptions de la norme IS-1 précitée.

Les figures 3 et 4 représentent plus en détail les bornes de connexion 12 et 14, qui sont structurellement identiques.

A la différence des connecteurs traditionnels où ces bornes assurent à la fois le raccordement électrique et la liaison mécanique entre sonde et connecteur de générateur par un système à vis, dans la présente invention ces bornes ont pour seule fonction d'assurer la liaison électrique entre les éléments de contact 16, 18 et les surfaces homologues 20, 22 de la sonde. A cet effet, l'élément de contact 16 de la borne 12 (et de même pour l'élément de contact 18 de l'autre borne 14) comporte une surface d'appui concave 30 de forme cylindrique, homologue de la forme cylindrique convexe de la surface 20 de manière à venir en appui sur cette dernière en épousant sa forme. L'élément de contact 16 est sollicité radialement par un élément élastique tel qu'un ressort 32 de manière à procurer une force d'appui suffisante pour que la résistance de contact entre l'élément 16 et la surface conductrice 20 soit la plus faible possible. Pour que la surface concave 30 se présente toujours dans la bonne direction par rapport à l'extrémité de la sonde, il est prévu des moyens de guidage tels qu'une cannelure 34 pour empêcher l'élément 16 de tourner dans son logement. L'ensemble est par exemple serti en 36 de manière à former une borne de contact monobloc, dépourvue notamment des bouchons de passage qui étaient nécessaires avec les bornes de contact de l'art antérieur. L'ensemble selon l'invention est donc parfaitement étanche et les éléments électriques sont mis à l'abri de tout risque de contact avec des fluides corporels.

Pour assurer le maintien mécanique de l'extrémité de sonde dans le connecteur du générateur, il est prévu selon l'invention une cale de blocage 38 (représentée de façon isolée figure 6) venant s'intercaler entre sonde et connecteur. Cette cale est réalisée en un matériau rigide, tel qu'une résine époxy, de sorte que son insertion entre le corps du connecteur de générateur (en matériau rigide tel qu'une résine époxy également) et la gaine de la sonde 28 (en matériau déformable tel qu'une résine silicone) va permettre une insertion à force et un maintien mécanique entre ces deux éléments du fait de l'élasticité de la partie de sonde légèrement déformée par la cale près du débouché du logement 26.

Plus précisément, la cale 38 présente, en coupe longitudinale, une forme biseautée, homologue de celle d'un évidement 40 (figure 5) formé dans le logement 26.

Plus précisément, la cale présente, intérieurement, une surface cylindrique 42 de même rayon que la surface extérieure cylindrique 46 du corps de sonde 28 de manière à venir épouser la forme de celle-ci, et une surface extérieure tronconique 44 homologue d'une surface intérieurement également tronconique et de même angle au sommet 48 de l'évidement 40 (figure 5).

Dans un plan radial, figure 2, la cale 38 s'étend circonférentiellement sur un secteur angulaire d'angle α, typiquement compris entre 90 et 120°. Cette caractéristique n'est cependant pas limitative et la cale peut par exemple s'étendre sur un secteur angulaire plus important, ou même sur la quasi-totalité de la circonférence, avec dans ce cas une forme de bague conique fendue.

Avantageusement, il est prévu des moyens d'encliquetage de la cale en fin de course, par exemple sous forme d'une gorge circonférentielle 50 (figures 1 et 6) coopérant avec un relief circonférentiel homologue 52 (figures 1 et 5) de la cavité 40.

Par ailleurs, pour empêcher que la cale ne sorte de l'évidement 40 en l'absence de sonde dans le logement 26, il est prévu des moyens de retenue, par exemple sous forme d'un ergot 54 susceptible de venir en butée contre le relief 52 lorsque la cale est dans sa position extrême sortie de l'évidement 40. Bien entendu, il est prévu une fente longitudinale 56 (figure 5) ménagée dans l'évidement 40 pour éviter que l'ergot 54 ne vienne empêcher un bon contact des surfaces coniques homologues 44 et 48 lorsque la cale est insérée à fond, en direction proximale, dans l'évidement 40.

On comprendra que, après l'insertion de la sonde au fond du logement 26, il suffit de pousser en direction proximale la cale 38 dans l'évidement 40 jusqu'à immobilisation et encliquetage. Cette cale va déformer légèrement l'arrière du connecteur de sonde, du fait du caractère élastique du matériau de la gaine, pour se mettre en place dans l'évidement. La cale peut être enfoncée dans l'évidement par le praticien au moment du montage du connecteur ; on peut également prévoir sur le conducteur de sonde un épaulement 58 placé à une distance telle que l'insertion de la sonde dans l'évidement 40 vienne en même temps pousser la cale, réalisant ainsi simultanément la mise en contact électrique et le verrouillage mécanique du connecteur de sonde sur le connecteur du générateur.

Pour permettre un démontage plus aisé, on peut prévoir un alvéole tel que 60 (figure 6) permettant de venir avec un outil approprié exercer sur la cale 38 une pression suffisante pour dégager la gorge 50 du relief 52 et ainsi débloquer le système de verrouillage.

## Revendications

1. Un dispositif médical implantable actif, notamment un stimulateur cardiaque, défibrillateur et/ou cardioverteur, comprenant un connecteur de générateur (10) comportant un logement femelle axial (26) recevant un connecteur de sonde (24), au moins une borne de liaison (12, 14) et des moyens d'immobilisation mécanique du connecteur de sonde dans le logement, la borne de liaison comportant des moyens pour exercer une pression d'appui radial d'un élément conducteur (16, 18) de la borne contre un élément conducteur (20, 22) du connecteur de sonde, ledit dispositif comprenant des moyens d'immobilisation distincts des bornes de liaison sous forme d'une cale de blocage (38) présentant en section longitudinale un profil cunéiforme, **caractérisé en ce que** ladite cale est propre à être insérée à force au débouché du logement entre une surface extérieure (46) du connecteur de sonde et une surface intérieure (48) du logement.

2. Le dispositif de la revendication 1, dans lequel la surface intérieure (48) du logement (26) comporte un évidement conique (40) dont la surface (48) forme une rampe homologue du profil cunéiforme de la cale de blocage.

3. Le dispositif de la revendication 1, dans lequel la cale de blocage comporte une surface cylindrique (42) propre à venir en contact avec la surface extérieure (46) de la sonde et une surface conique (44) propre à venir en contact avec la surface intérieure (48) du logement.

4. Le dispositif de la revendication 1, dans lequel, dans un plan radial, la cale (38) s'étend circonférentiellement sur un secteur angulaire d'ouverture (α) comprise entre 90 et 120°.

5. Le dispositif de la revendication 1, comprenant en outre des moyens (50, 52) d'encliquetage en position opérante de la cale dans le logement.

6. Le dispositif de la revendication 5, dans lequel les moyens d'encliquetage comprennent une gorge (50) formée dans la cale coopérant avec un relief homologue (52) formé dans le logement, ou l'inverse, gorge et relief s'étendant sur une fraction de circonférence dans un plan radial.

7. Le dispositif de la revendication 1, comportant des moyens (52, 54) de retenue de la cale de blocage dans le logement en l'absence de sonde.

8. Le dispositif de la revendication 7, dans lequel les moyens de retenue comportent un ergot (54) de butée en position proximale, coopérant avec un relief (52) du logement en position distale.

9. Le dispositif de la revendication 1, dans lequel les moyens de la borne de liaison pour exercer une pression d'appui radial comportent des moyens (32) de sollicitation élastique de l'élément conducteur (16, 18) de la borne contre l'élément conducteur (20, 22) du connecteur.

10. Le dispositif de la revendication 1, dans lequel la sonde comporte un épaulement (58) pour solliciter axialement la cale de blocage dans le sens de l'enfoncement lors de l'insertion de la sonde dans le logement.

## Patentansprüche

1. Aktive implantierbare medizinische Vorrichtung, insbesondere ein Herzstimulator, Defibrillator und/oder Kardiovertierer, umfassend eine Anschlussklemme des Generators (10), die einen axialen weiblichen Aufnahmeraum (26), der ein Verbindungsstück für eine Sonde (24) aufnimmt, mindestens eine Anschlussklemme (12, 14) und Mittel zur mechanischen Verriegelung des Verbindungsstücks für eine Sonde in dem Aufnahmeraum aufweist, wobei die Anschlussklemme Mittel zum Ausüben eines radialen Auflagedrucks eines leitenden Elements (16, 18) der Klemme gegen ein leitendes Element (20, 22) des Verbindungsstücks für eine Sonde,
**dadurch gekennzeichnet,**
**dass** die Vorrichtung von den Anschlussklemmen verschiedene Mittel zur Verriegelung umfasst, in Form eines Blockierungskeils (38), der im Längsschnitt ein keilförmiges Profil aufweist, wobei der Keil geeignet ist, mit Kraft am Ausgang des Aufnahmeraums zwischen eine äußere Oberfläche (46) des Verbindungsstücks für eine Sonde und eine innere Oberfläche (48) des Aufnahmeraums eingeführt zu werden.

2. Vorrichtung gemäß Anspruch 1, in welcher die innere Oberfläche (48) des Aufnahmeraums (26) eine konische Vertiefung (40) aufweist, deren Oberfläche (48) eine schiefe Ebene bildet, homolog zu dem keilförmigen Profil des Blockierunaskeils.

3. Vorrichtung gemäß Anspruch 1, in welcher der Blockierungskeil eine zylindrische Oberfläche (42) aufweist, die geeignet ist, mit der äußeren Oberfläche (46) der Sonde in Kontakt zu kommen, und eine konische Oberfläche (44), die geeignet ist, mit der inneren Oberfläche (48) des Aufnahmeraums in Kontakt zu kommen.

4. Vorrichtung gemäß Anspruch 1, in welcher, in einer Radialebene, der Keil (38) sich über einen Kreisausschnitt mit Öffnungswinkel (α) zwischen 90 und 120° erstreckt.

5. Vorrichtung gemäß Anspruch 1, ferner umfassend Mittel (50, 52) zur Sperrung des Keils in dem Aufnahmeraum in Wirkposition.

6. Vorrichtung gemäß Anspruch 5, in welchem die Mittel zur Sperrung eine Vertiefung (50) aufweisen, die auf dem Keil gebildet ist, die mit einer homologen Erhebung (52), die in dem Aufnahmeraum gebildet ist, zusammenwirkt, oder umgekehrt, wobei die Vertiefung und die Erhebung sich über einen Bruchteil des Umfangs auf einer Radialebene erstrecken.

7. Vorrichtung gemäß Anspruch 1, die Mittel (52, 54) zum Zurückhalten des Blockierungskeils in dem Aufnahmeraum aufweist, in Abwesenheit einer Sonde.

8. Vorrichtung gemäß Anspruch 7, in welcher die Mittel zum Zurückhalten einen Vorsprung zum Stoppen (54) in proximaler Position aufweisen, der mit einer Erhebung (52) des Aufnahmeraums in distaler Position zusammenwirkt.

9. Vorrichtung gemäß Anspruch 1, in welcher die Mittel der Anschlussklemme zur Ausübung eines radialen Auflagedrucks Mittel (32) zur elastischen Belastung des leitenden Elements (16, 18) der Klemme gegen das leitende Element (20, 22) des Verbindungsstücks aufweisen.

10. Vorrichtung gemäß Anspruch 1, in welcher die Sonde einen Vorsprung (58) aufweist, um den Blockierungskeil axial zu belasten, im Sinne eines Eintreibens, bei Einführen der Sonde in den Aufnahmeraum.

## Claims

1. An active implantable medical device, in particular a pacemaker, defibrillator and/or cardioverter, comprising a generator connector (10) including an axial female cavity (26) receiving a probe connector (24), at least one connection terminal (12, 14), and a means of mechanical immobilisation of the probe connector in the cavity, the connection terminal comprising means to exert a radial support pressure on a conducting element (16, 18) of the terminal against a conducting element (20, 22) of the probe connector,
said device including immobilisation means, distinct from the connection terminals in the form of a locking wedge (38) having in longitudinal cross-section a cuneiform profile, **characterised in that** said wedge is adapted to be inserted by a force fit at the outlet of the cavity between an external surface (46) of the probe connector and an internal surface (48) of the cavity.

2. The device of claim 1, wherein the internal surface (48) of the cavity (26) has a conical recess (40) whose surface (48) forms a slope complementary to the cuneiform profile of the locking wedge.

3. The device of claim 1, wherein the locking wedge has a cylindrical surface (42) configured to contact the external surface (46) of the probe and a conical surface (44) configured to contact the internal surface (48) of the cavity.

4. The device of claim 1, in which, in a radial plane, the wedge (38) spreads circumferentially over an angular sector having an aperture (α) comprised between 90 and 120°.

5. The device of claim 1, further comprising means (50, 52) for interlocking the wedge in an operating position in the cavity.

6. The device of claim 5, wherein the interlocking means comprises a groove (50) formed in the wedge cooperating with a complementary protrusion (52) formed in the cavity, or vice versa, wherein the groove and the protrusion spread over a portion of the circumference in a radial plane.

7. The device of claim 1, comprising means (52, 54) for retention of the locking wedge in the cavity in the absence of a probe.

8. The device of claim 7, wherein the retention means comprises a stop spur (54) in a proximal position, cooperating with a protrusion (52) of the cavity in a distal position.

9. The device of claim 1, wherein the means of the connection terminal for exerting a radial support pressure includes means (32) for elastically urging the conducting element (16, 18) of the terminal against the conducting element (20, 22) of the connector.

10. The device of claim 1, wherein the probe comprises a shoulder (58) for axially urging the locking wedge in the direction of force-fitting during the insertion of the probe in the cavity.
